# EUROPEAN PATENT APPLICATION

(11) **EP 3 015 462 A1**
(43) Date of publication of application: **04.05.2016**
(21) Application number: 14190778.2
(22) Date of filing: 29.10.2014
(51) Int. Cl.: C07D 255/02, A61P 35/00, A61K 51/08

(54) **Enantiopure chelating agents for chelator coupled pharmaceuticals, corresponding preparation method thereof and chelator coupled pharmaceuticals**

(71) Applicant: OctreoPharm Schiences GmbH, 13125 Berlin (DE); Chematech, 21000 Dijon (FR)
(72) Inventor: Boschetti, Frédéric, Dr., 21800 Chevigny-Saint-Sauveur (FR); Bouterfa, Hakim, Dr., 97265 Würzburg (DE); Kaufmann, Jens, Dr., 13507 Berlin (DE)
(74) Representative: Gulde & Partner

(57) **Abstract**

According to the present invention an enantiopure chelating compound of general Formula 10A or 10B is provided: 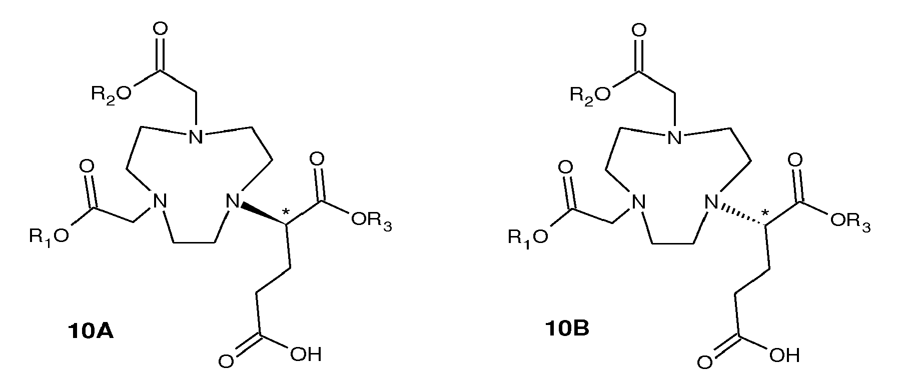 wherein each of R₁ through R₃ is independently selected hydrogen, substituted or unsubstituted C₁-C₂₀ alkyl, substituted or unsubstituted C₂-C₂₀ alkenyl, substituted or unsubstituted C₂-C₂₀ alkynyl, substituted or unsubstituted C₃-C₁₀ cycloalkyl, substituted or unsubstituted C₆-C₆₀ aryl, or -Si(R₄)(R₅)(R₆), and each of R₄ through R₆ is independently selected a substituted or unsubstituted group consisting of C₁-C₂₀ alkyl, C₂-C₂₀ alkenyl, C₂-C₂₀ alkynyl, C₁-C₂₀ alkoxy, C₃-C₁₀ cycloalkyl, C₆-C₆₀ aryl, and C₆-C₆₀ aryloxy.

The present invention further refers to a corresponding preparation method of the chelating compound and chelator coupled pharmaceuticals including the same.

## Description

The present invention refers to an enantiopure chelating compound, a complex comprising said chelating compound as well as pharmaceutical compositions comprising the chelating compound, respectively the complex. The present invention further provides a preparation method for the chelating compound und refers to the use of the chelating compound, respectively the complex for preparing a chelator coupled pharmaceutical. In addition, the present invention also refers to chelator coupled pharmaceuticals, especially peptide conjugates, for example SRIF agonist or antagonist.

### Technical Background of the Invention

The present invention relates to the field of chelator coupled pharmaceuticals and their use in nuclear medicine as tracers, imaging agents and for the treatment of various disease states (radiopharmaceuticals). It is well known that tumors may express unique proteins associated with their malignant phenotype or may over-express constituent proteins in greater number than normal cells. The expression of distinct receptors on the surface of tumor cells offers the opportunity to diagnose and characterize disease by probing the phenotypic identity and biochemical composition and activity of the tumor. Radioactive molecules that selectively bind to specific tumor cell surface proteins provide an attractive route for imaging and treating tumors under non-invasive conditions. Important examples are analogues of CA9 (carbonic anhydrase 9) or SRIF1 (somatostatin release inhibiting factor) which are being used in the treatment of tumors. A development has been the use of radiolabeled versions of regulatory peptide analogues in the in vivo localization and treatment of tumors. For example, a radiopharmaceutical based on diethylenetriaminepentaacetic acid (DTPA) as chelating agent labeled with ¹¹¹In and being bound to somatostatin analogue octreotide (-Phe-Cys-Phe-D-Trp-Lys-Thr-Cys-Thr(ol) (disulfide bond)) is commercially available under the trade name OctreoScan.

Several alternative chelating agents for radiopharmaceuticals have been developed (for example K.-P. Eisenwiener et al., A Convenient Synthesis of Novel Bifunctional Prochelators for Coupling to Bioactive Peptides for Radiometal Labelling, Bioorganic & Medicinal Chemistry Letters, 10 (2000), pages 2133-2135). In particular, K.-P. Eisenwiener et al. developed a non-stereoselective 5-step synthesis of [1-(1-carboxy-3-carbo-tert-butoxypropyl)-4,7-(carbo-tert-butoxymethyl)-1,4,7-triazacyclononane (NODAGA(tBu)₃)]: 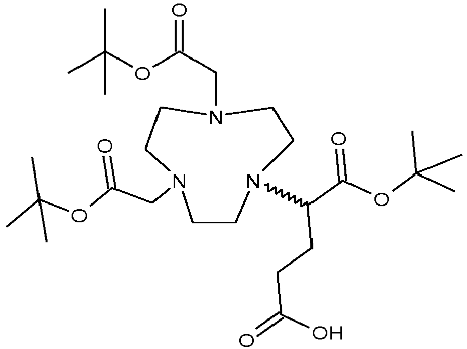 NODAGA(tBu)₃ was coupled to octreotide and labeled with the radiometals ¹¹¹In and ⁶⁷Ga. The peptide conjugate has been shown to be useful to visualize primary tumors and metastases which express somatostatin receptors subtype 2 (sstr2), such as neuroendocrine tumors *(*NODAGATOC, a New Chelator-Coupled Somatostatin Analogue Labeled with [67/68Ga] and [111In] for SPECT, PET, and Targeted Therapeutic Applications of Somatostatin Receptor (hsst2) Expressing Tumors, Bioconjugate Chem., 2002, 13, pages 530-541).

Certain drugs, for example thalidomide, have been shown to have differing safety and/or efficacy profiles depending on whether the drug is an enantiomeric mixture of isomers, or is an optically pure or optically enriched isomer composition. Thus, methods of preparing purified optically enriched or optically pure chelating agents have recently been developed. In particular, WO 2005/001415 A2 and S.G. Levy et al. (Development of Multigram Asymmetric Synthesis of 2-(R)-2-(4,7,10-Tris tert-Butylcarboxymethyl-1,4,7,10-tetraazacyclododec-1-yl)-pentanedioic Acid, 1-tert-Butyl Ester, (R)-tert-Bu4-DOTAGA, Organic Process Research & Development, 2009, 13, pages 535-542) describe a synthetic approach using a specific chiral glutaric acid derivate as a synthetic building block which is bound to a 1,4,7,10-tetraazadodecane-1,4,7,10 tetraacetic acid (DOTA) derivative leading to an enantiopure chelating DOTA-compound:

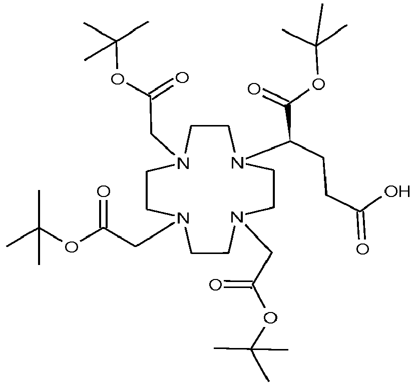

Recently, a first study on enantiopure radiopharmaceuticals has been published by A. N. Singh et al. (Enantiopure bifunctional chelators for copper radiopharmaceuticals - Does chirality matter in radiotracer design?, European Journal of Medicinal Chemistry, 80 (2014), pages 308-315). However, the investigated forms of R,R-, S,S-, and R,S-peptide conjugates showed similar biological behavior. Hence, whether or not specific enantiomers of peptide conjugates show similar biological behavior is not predictable and must be examined for each single case.

### Summary of Invention

According to the present invention an enantiopure chelating compound of general Formula 10A or 10B is provided: 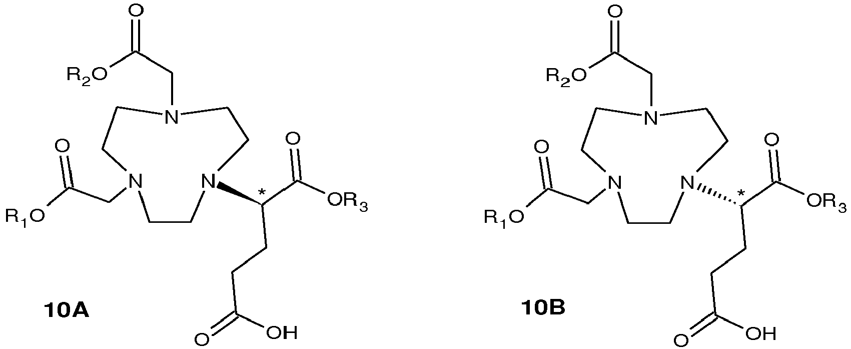 wherein:
each of R₁ through R₃ is independently selected hydrogen, substituted or unsubstituted C₁-C₂₀ alkyl, substituted or unsubstituted C₂-C₂₀ alkenyl, substituted or unsubstituted C₂-C₂₀ alkynyl, substituted or unsubstituted C₃-C₁₀ cycloalkyl, substituted or unsubstituted C₆-C₆₀ aryl, or -Si(R₄)(R₅)(R₆), and
each of R₄ through R₆ is independently selected a substituted or unsubstituted group consisting of C₁-C₂₀ alkyl, C₂-C₂₀ alkenyl, C₂-C₂₀ alkynyl, C₁-C₂₀ alkoxy, C₃-C₁₀ cycloalkyl, C₆-C₆₀ aryl, and C₆-C₆₀ aryloxy.

For example, in Formulae 10A or 10B, each of R₁ to R₃ may be independently selected hydrogen, substituted or unsubstituted C₁-C₁₀ alkyl, or substituted or unsubstituted C₆-C₁₄ aryl. Particularly, in Formulae 10A or 10B, each of R₁ to R₃ may be hydrogen, methyl, t-butyl, or benzyl.

According to one embodiment, the enantiopure chelating compound is a compound represented by one of compounds 6A (1-(R)-(1-carboxy-3-carbo-tert-butoxypropyl)-4,7-(carbo-tert-butoxymethyl)-1,4,7-triazacyclo-nonane or 6B (1-(S)-(1-carboxy-3-carbo-tert-butoxypropyl)-4,7-(carbo-tert-butoxymethyl)-1,4,7-triazacyclo-nonane in short R-NODAGA(tBu)₃ or S-NODAGA(tBu)₃):

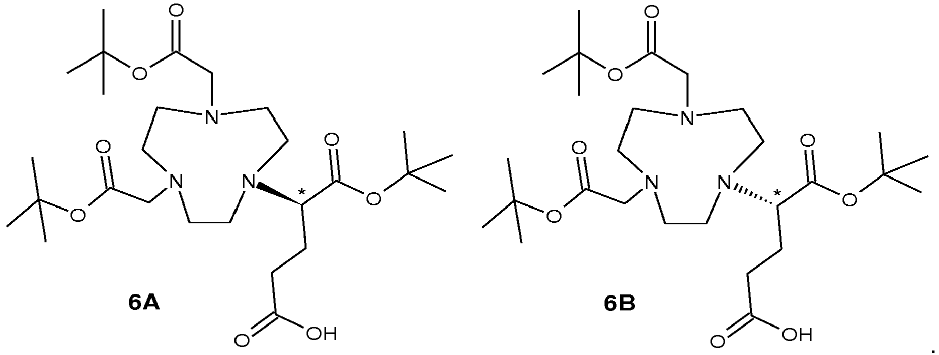

According to another embodiment, the enantiopure chelating compound is a compound represented by one of compounds 7A or 7B:

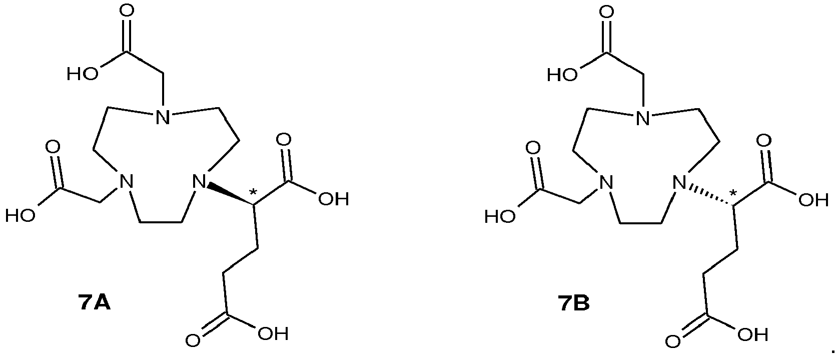

The invention further refers to a complex comprising one of the above mentioned chelating compounds and an element selected from the group consisting of Re, Al, In, As, Y, Cu, Tc, Er, Sn, Te, Pr, Ga, Pt, At, Au, Tb, Pd, Dy, Pm, Sm, Gd, Ho, Tm, Yb, Lu, Rh, Ag, F and I. The element may be a radionuclide, especially selected from the group consisting of ¹⁸⁶Re, ¹⁸⁸Re, ¹¹¹In, ¹¹³In, ⁷¹As, , ⁹⁰Y, ⁶⁷Cu, ⁶⁴Cu, ⁹⁹Tc, ¹⁶⁹Er, ¹²¹Sn, ¹²⁷Te, ¹⁴²Pr, ¹⁴³Pr, ⁶⁶Ga, ⁶⁷Ga, ⁶⁸Ga, ⁷²Ga, ¹⁹⁵Pt, ²¹¹At, ¹⁹⁸Au, ¹⁹⁹Au, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹⁴Ag, ¹⁸F, ¹²⁴I and ¹³¹I.

Based on the enantiopure chelating compounds, enantiopure chelator coupled pharmaceuticals can be prepared. Use of enantiopure chelating compounds enhances safety of the respective pharmaceuticals, i.e. any risk with respect to an undesirable departing efficacy profile of the other enantiomer could be avoided.

Further, it has been found that the enantiopure chelating compounds, especially compounds 6A and 6B, show equivalent biological behavior compared to the known racemic mixture of the chelating agent. In particular, the two diastereomeres (R)-Nodaga-JR11 and (S)-Nodaga-JR11 prepared by use of enantiopure chelating compounds 6A and 6B showed comparable IC50 receptor affinity as the mixture (R/S)-Nodaga-JR11 prepared from the racemic mixture of NODAGA(tBu)₃ in in-vitro sst-receptor binding assays. Furthermore the individual diastereomeres as well as the product mixture exhibit the same toxicological profile as shown in extended single dose studies in wistar rats.

In addition, the conventional manufacturing of these conjugates with racemic starting material results in the formation of a racemate or diastereomeric product. Purification of the later is a cost intensive procedure and results in poor product yields. The employment of enantiomerically enriched or pure starting material produces high product purity. Furthermore, the registration pathway to gain marketing authorization of a product mixture involves extensive proof of pharmacological and toxicological analogy of the individual compounds.

Another aspect of the invention thus refers to a pharmaceutical formulation comprising the above mentioned chelating compound or complex and a pharmaceutically acceptable carrier. The chelating compound or complex may be bound to small pharmacophores, peptides, antibodies or their fragments and nanoparticles.

The term 'pharmaceutically-acceptable carrier' as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be compatible with the other ingredients of the formulation and, of course, not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; ethyl alcohol; pH buffered solutions; polyesters, polycarbonates; and polyanhydrides.

In particular, according to another aspect of the present invention there is provided a chelator coupled pharmaceutical including the chelating compound or a complex thereof being covalently coupled to a compound bearing a pharmacophore structure. A pharmacophore is an abstract description of molecular features which are necessary for molecular recognition of a ligand by a biological macromolecule. A pharmacophore is defined to be an ensemble of steric and electronic features that is necessary to ensure the optimal supramolecular interactions with a specific biological target and to trigger or block its biological response. The pharmacophore may be for example part of a peptide, antibodies or their fragments and nanoparticles.

Preferably, the chelator pharmaceutical is a peptide conjugate of a peptide agonist or peptide antagonist, especially a SRIF antagonist, with the chelating compound, respectively a complex thereof. In particular, the SRIF antagonist may bind to SRIF receptors SSTR1-5, especially SSTR2. The peptide antagonist will be labeled with an isotope by the chelating compound.

Thus, the present invention relates also to the use of a chelating compound, respectively a complex thereof for preparing a chelator coupled pharmaceutical, especially for preparing a peptide conjugate radiopharmaceutical by coupling of the chelating agent to a peptide antagonist, especially a SRIF antagonist.

Finally, there is provided a method of preparing chelating compounds 10A and 10B including the step of:
conversion of compound 9A or 9B with compound 8 and subsequent remove of the benzyl protective moiety according to the following reaction scheme:

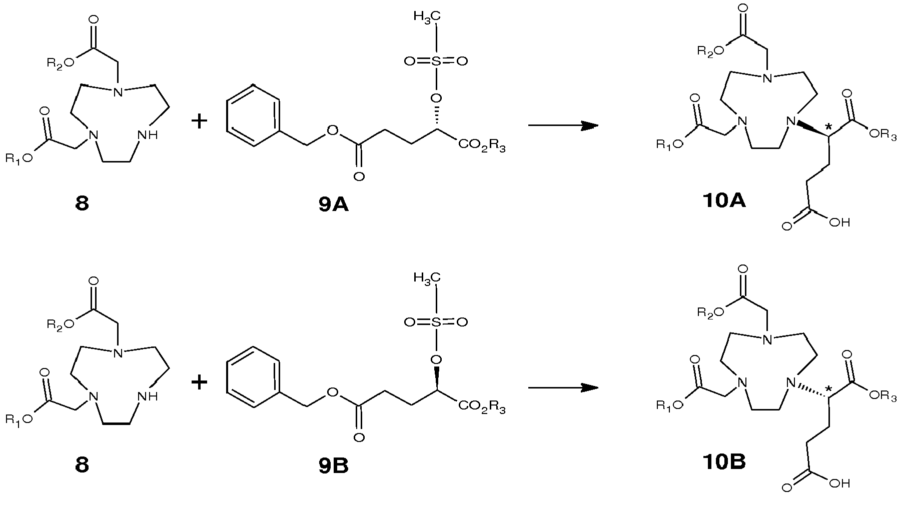

### Detailed Description of the Invention

In the following, a general approach to an enantiopure chelating compound of general Formula 10A or 10B will be presented: 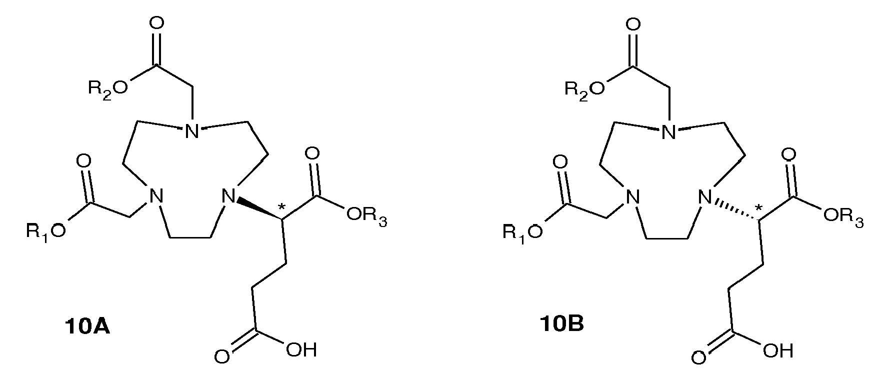
each of R₁ through R₃ is independently selected hydrogen, substituted or unsubstituted C₁-C₂₀ alkyl, substituted or unsubstituted C₂-C₂₀ alkenyl, substituted or unsubstituted C₂-C₂₀ alkynyl, substituted or unsubstituted C₃-C₁₀ cycloalkyl, substituted or unsubstituted C₆-C₆₀ aryl, or -Si(R₄)(R₅)(R₆), and
each of R₄ through R₆ is independently selected a substituted or unsubstituted group consisting of C₁-C₂₀ alkyl, C₂-C₂₀ alkenyl, C₂-C₂₀ alkynyl, C₁-C₂₀ alkoxy, C₃-C₁₀ cycloalkyl, C₆-C₆₀ aryl, and C₆-C₆₀ aryloxy.
wherein:

Further, distinct embodiments for chelating compounds falling under general Formula 10A or 10B are provided. Based on two specific chelating compounds chelator coupled pharmaceuticals, in particular peptide conjugates of a SRIF antagonist, have been prepared and evaluated.

### Definitions

As described herein, compounds of the invention may be optionally substituted with one or more substituents. In general, the term "substituted" means that a hydrogen radical of the designated moiety is replaced with the radical of a specified substituent, provided that the substitution results in a stable or chemically feasible compound. More than one position in any given structure may be substituted with a substituent, the substituent may be either the same or different at every position. Combinations of substituents envisioned by this invention are preferably those that result in the formation of stable or chemically feasible compounds. A stable compound or chemically feasible compound is one in which the chemical structure is not substantially altered when kept at a temperature from -80° C to +50° C, in the absence of moisture or other chemically reactive conditions, for at least a week, or a compound which maintains its integrity long enough to be useful for diagnostic or therapeutic administration to a patient. Exemplary substituents include halides such as F, Cl, Br, and I; hydroxyl groups, C₁-C₁₀ alkyl groups, C₁-C₁₀ alkoxy groups, and C_{6-C10} aryl groups.

As used herein, the term "independently selected" means that the same or different values may be selected for multiple instances of a given variable in a single compound.

The term "alkyl", used alone or as part of a larger moiety, refers to an optionally substituted straight or branched chain hydrocarbon group.

The term "alkenyl", used alone or as part of a larger moiety, refers to an optionally substituted straight or branched chain hydrocarbon group having at least one double bond.

The term "alkynyl", used alone or as part of a larger moiety, refers to an optionally substituted straight or branched chain hydrocarbon group having at least one triple bond.

The term "cycloalkyl" used alone or as part of a larger moiety, refers to an optionally substituted saturated ring system of 3 to 10 ring carbon atoms. Exemplary monocyclic cycloalkyl rings include cyclopropyl, cyclobutyl, cyclopentyl, cyclohexyl, and cycloheptyl.

The terms "aryl" or "aryloxy" used alone or as part of a larger moiety, refer to an optionally substituted C₆₋₆₀ aromatic hydrocarbon moiety comprising at least one aromatic ring. Preferably, the aryl group is a C₆₋₁₀ aryl group. In case of an aryloxy group the moiety is bound to the structure though an oxygen atom. Exemplary aryl groups include phenyl, naphthyl, or anthracenyl.

The term "alkoxy" used as part of a larger moiety, refers to optionally substituted ethers of a straight or branched chain hydrocarbon group.

### Synthesis

In the following, an exemplary embodiment of the present invention will be described.

In a first step of the synthetic approach to chiral compound 6A chiral glutaric acid derivate 3A is prepared according to the following reaction scheme (cf. WO 2005/00145 A2):

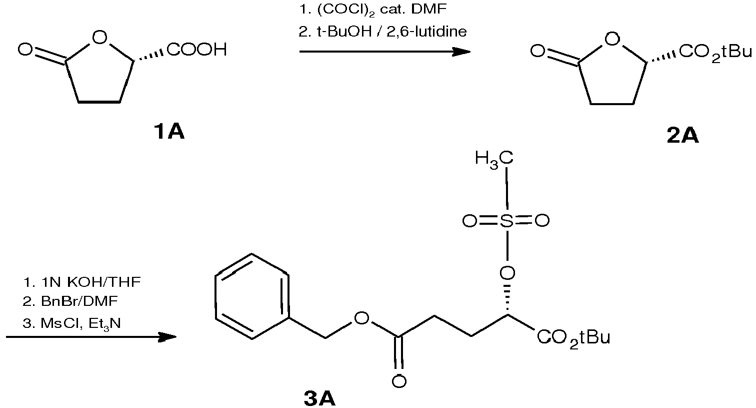

In a second step of the synthetic approach compound 3A and compound 4 (commercially available at CheMatech SAS, France) are coupled to each other to form compound 5A. Afterwards the protective benzyl group is removed according to the following reaction scheme:

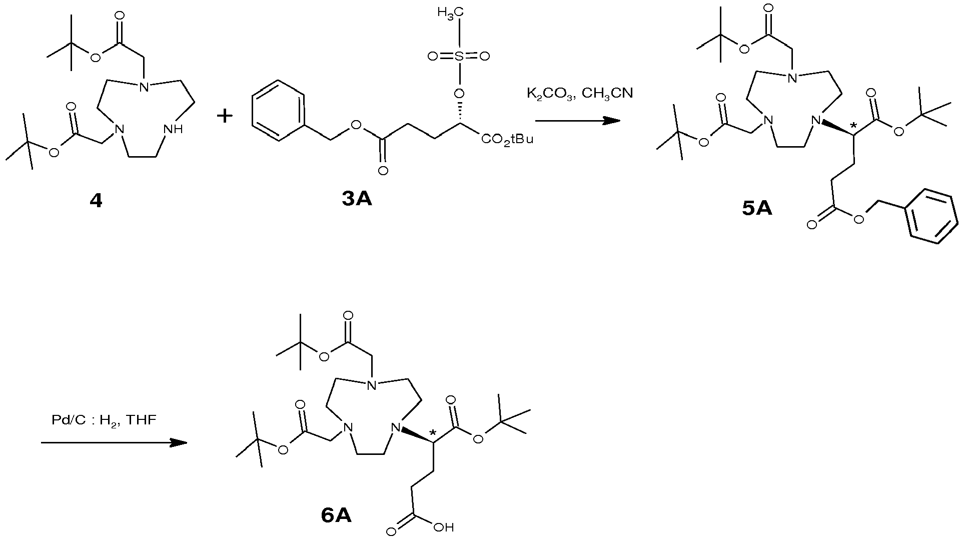

### Synthesis of Compound 2A

70.0 g (0.538 mol) of compound 1A is dissolved in 315 ml of CH₂Cl₂. The solution is cooled and kept at 3°C under N₂ atmosphere. 170.7 g (1.345 mol) of oxalyl chloride is slowly added afterwards 0.80 ml of DMF is carefully added to the solution. After completion of the addition, the mixture is warmed to room temperature and stirred for 2h. Then, the solvent is evaporated under reduced pressure. The remaining material is dissolved in 15 ml of CH₂Cl₂ and the solvent is evaporated once again. 69.2 g (0.646 mol) of 2,6-lutidine and 79.8 g (1.076 mol) of t-BuOH are dissolved in 350 ml of CH₂Cl₂. The mixture is cooled to 3°C. Then a solution of acyl chloride previously synthesized in 50 ml of CH₂Cl₂ is added dropwise over a period of 10 min. At the end of the addition, the mixture is warmed to room temperature and stirred for 12 h. Solvent is evaporated. The remaining oil is dissolved in 500 ml of ethyl acetate and purified on Silica plug (around 2L of ethyl acetate). The solution is concentrated and treated with charcoal during 30 min. After filtration on celite, the solvent is evaporated under reduced pressure to obtain a yellow solution which is kept for recrystallization to obtain compound 2A as pale yellow powder.

### Synthesis of Compound 3A

A solution of 1 N KOH is added to a solution of compound 2A dissolved in 350 ml of THF. The solution is stirred at 40°C for 2h. Solvent is evaporated under reduced pressure to obtain a yellow oil which is dissolved in 900 ml of DMF. 83.3 g (0.487 mol) of benzyl bromide is added and the mixture is stirred at room temperature for 8h. The mixture is pooled on water at 2°C and the compound is extracted with ethyl acetate. After drying on MgSO₄, the organic phase is evaporated to obtain a yellow oil which is purified by flash chromatography (Silica gel; Hept/AcEt from 100/0 to 25/75). The compound previously obtained is dissolved in 975 ml of CH₂Cl₂ and the mixture is kept at 3°C. 39.4 g (0.390 mol) of triethylamine then 37.9 g (0.331 mol) methane sulfonyl chloride are added. The mixture is stirred at room temperature for 1 h. The mixture is washed with water, brine and the organic phase is evaporated to yield to compound 3A (108.9 g, yield 54%) which is used for the next step without further purification.

### Synthesis of Compound 5A

To a solution of 70.0 g (0.196 mol) of compound 4 and 81.2 g (0.587 mol) of K₂CO₃ in 1.4 l of CH₃CN is added 75.1 g (0.202 mol) of compound 3A. The mixture is stirred for 72 h at 50°C then cooled to room temperature. K₂CO₃ is removed by filtration and the solvent is evaporated. Compound 5A is obtained as a yellow oil which is used without further purification in the next step.

### Synthesis of Chelating Compound 6A - R-NODAGA(tBu)₃

Compound 5A is dissolved in 1.2 l of THF and 2 ml of water. 0.6 g of 10% Pd/C is added and the solution is placed under H₂ atmosphere and stirred for 12 h. The mixture is filtered on celite and THF is removed under reduced pressure. The remaining oil is purified by flash chromatography (Silica ; MeOH/NH₃) to obtain white foam (73.4 g ; yield 69 %).

HPLC: reverse phase 5u 4.6x250mm Lux Cellulose-4, mobile phase A) 20mM ammonium bicarbonate B) acetonitrile, A:B 70:30, modifier 0.1% diethylamine, diluent ethanol, flow rate 1ml/min, column temperature ambient, detection 210nm, injecting probe 2µl of 9.5 mg/ml, retention time 33.10 min

### Synthesis of Chelating Compound 6B - S-NODAGA(tBu)₃

Chelating compound 6B (S-NODAGA(tBu)₃) can be prepared in analogue way to the preparation of compound 6A but starting from commercially available glutaric acid derivate 1 B. The procedure can be summarized according to the following reaction scheme:

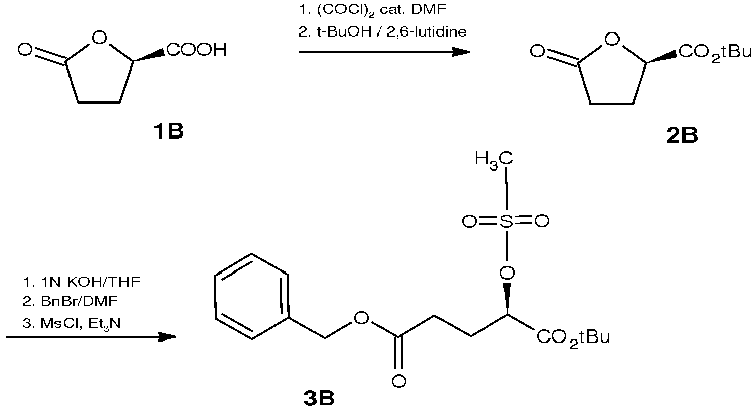

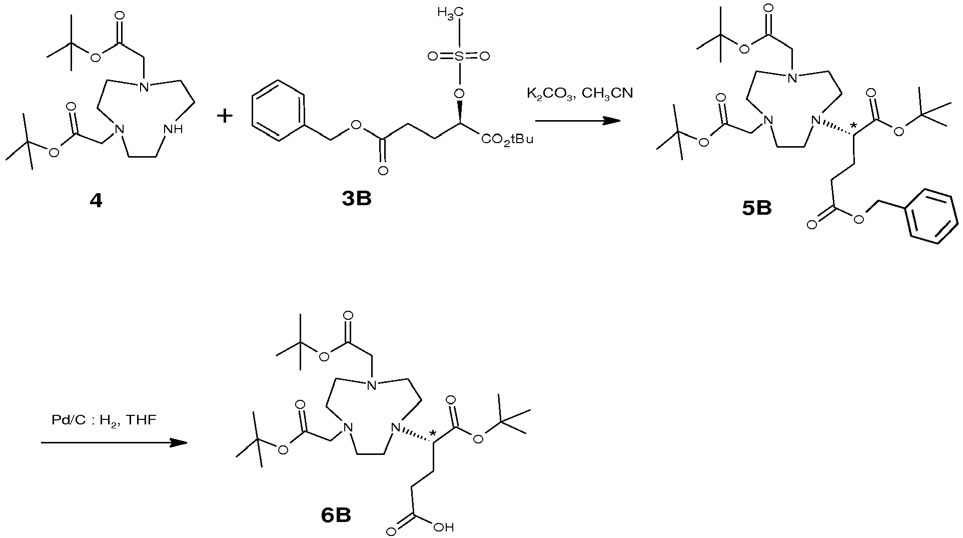

HPLC: reverse phase 5u 4.6x250mm Lux Cellulose-4, mobile phase A) 20mM ammonium bicarbonate B) acetonitrile, A:B 70:30, modifier 0.1% diethylamine, diluent ethanol, flow rate 1ml/min, column temperature ambient, detection 210nm, injecting probe 2µl of 9.5 mg/ml, retention time 28.50 min

### Synthesis of Chelating Compounds 7A and 7B

Removal of the BOC group of compounds 6A and 6B can be accomplished with strong acids such as trifluoroacetic acid neat or in dichloromethane, or with HCl in methanol.

### General Synthetic Approach to Chelating Compounds 10A and 10B

Based on the foregoing specific approach to compounds 6A and 6B, a more general synthetic approach the enantiopure NODAGA chelating compounds 10A and 10B could be accomplished.

In a first step, chiral compounds 11 A and 11 B could be achieved by esterifying glutaric acid derivatives 1A or 1 B in conventional manner:

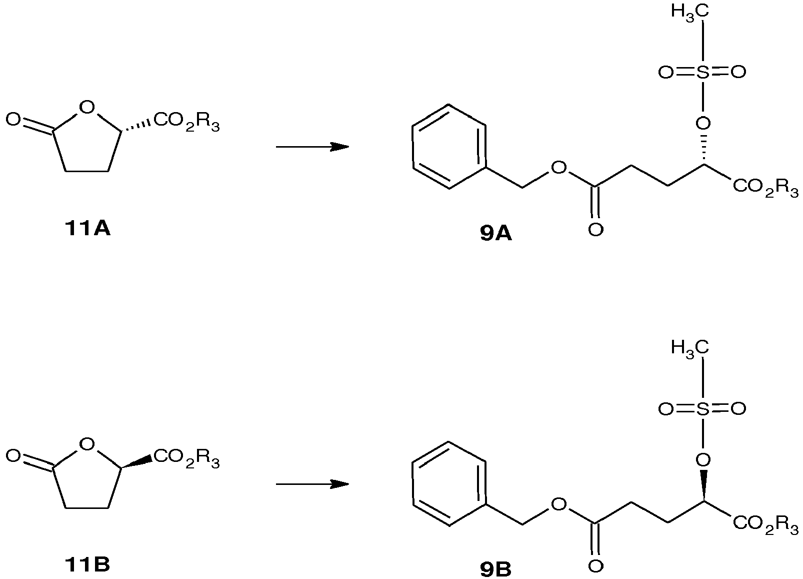

In a second step, compounds 9A or 9B are converted with compound 8. Compound 8 may be prepared in an analogue way to the preparation method of compound 4. Subsequently, the benzyl protective moiety is removed:

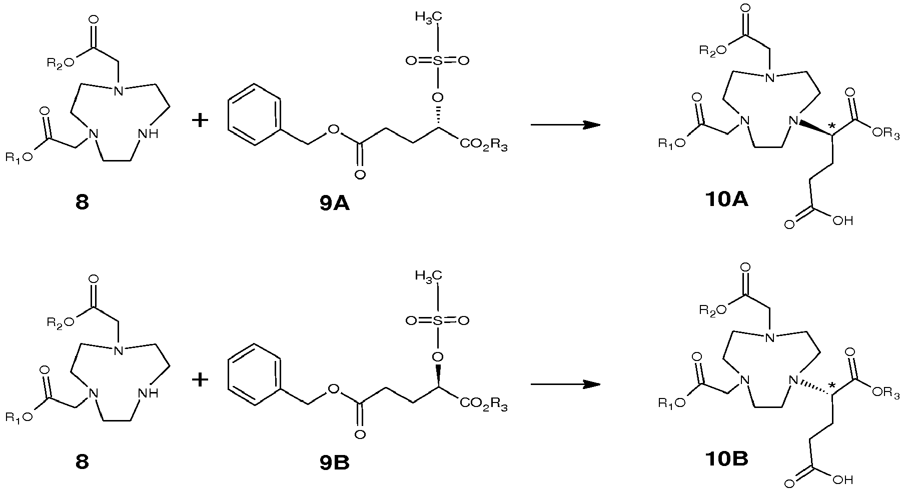

### Synthesis of R-NODAGA(tBu)₃-JR11 and S-NODAGA(tBu)₃-JR11

R-Nodaga(tBu)₃-JR11 [chemical structure R-NODAGA(tBu)₃-Cpa-c[D-Cys-Aph(Hor)-D-Aph(Cbm)-Lys-Thr-Cys]-D-Tyr-NH₂] and the corresponding S-Nodaga(tBu)₃-JR11 were synthesized following standard solid-phase peptide synthesis on a resin as described previously in Fani et al., J. Nucl. Med., Vol 53 (2012), pages 1481-1489 (cf. especially page 1484, left column, section *Synthesis of Analogs, Coupling to Chelators and Radiometals)* and Cescato et al., J. Med. Chem., 51 (2008), pages 4030-4037 (see especially page 4034, *Experimental Section).*

The evaluation of the biological behavior of the two diastereomeres R-Nodaga(tBu)₃-JR11 and S-Nodaga(tBu)₃-JR11 prepared by use of enantiopure chelating compound 6A, respectively 6B showed comparable IC50 receptor affinity as the mixture R/S-Nodaga(tBu)₃-JR11 prepared from the racemic mixture of NODAGA(tBu)₃ in in-vitro sst-receptor binding assays. Furthermore the individual diastereomeres as well as the product mixture exhibit the same toxicological profile as shown in extended single dose studies in wistar rats.

## Claims

1. An enantiopure chelating compound of general Formula 10A or 10B: 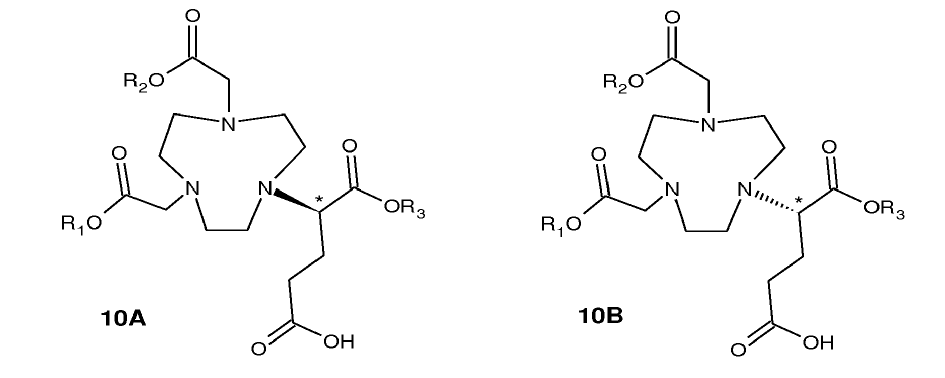 wherein:
each of R₁ through R₃ is independently selected hydrogen, substituted or unsubstituted C₁-C₂₀ alkyl, substituted or unsubstituted C₂-C₂₀ alkenyl, substituted or unsubstituted C₂-C₂₀ alkynyl, substituted or unsubstituted C₃-C₁₀ cycloalkyl, substituted or unsubstituted C₆-C₆₀ aryl, or -Si(R₄)(R₅)(R₆), and
each of R₄ through R₆ is independently selected a substituted or unsubstituted group consisting of C₁-C₂₀ alkyl, C₂-C₂₀ alkenyl, C₂-C₂₀ alkynyl, C₁-C₂₀ alkoxy, C₃-C₁₀ cycloalkyl, C₆-C₆₀aryl, and C₆-C₆₀aryloxy.

2. The enantiopure chelating compound of claim 1, wherein R₁ to R₃ is independently selected hydrogen, methyl, t-butyl, or benzyl.

3. The enantiopure chelating compound of claim 1, wherein the enantiopure chelating compound is a compound represented by one of compounds 6A and 6B:

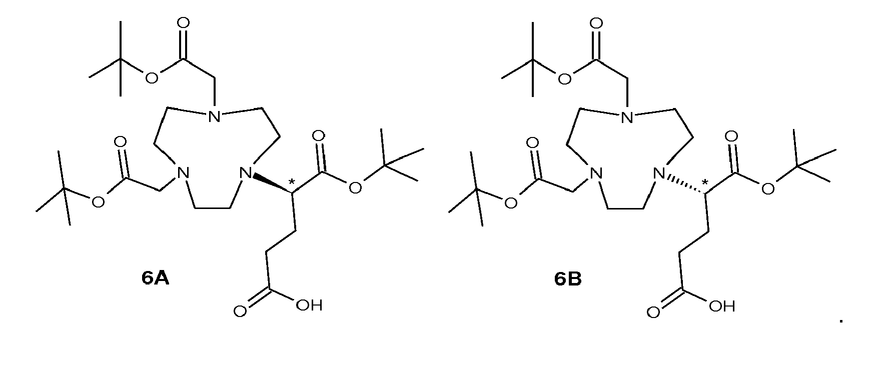

4. The enantiopure chelating compound of claim 1, wherein the enantiopure chelating compound is a compound represented by one of compounds 7A and 7B:

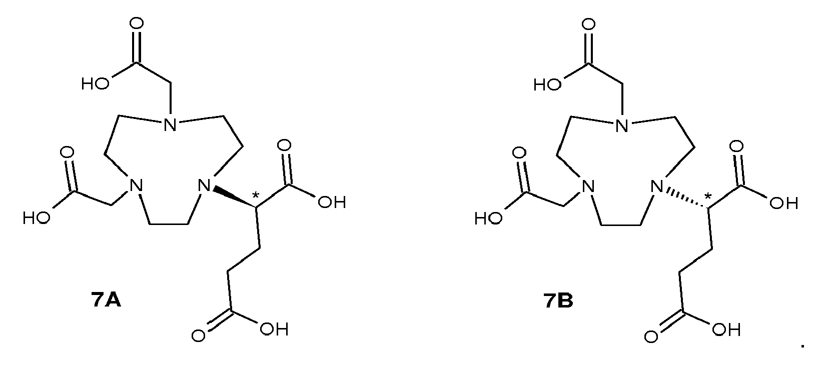

5. A complex comprising a chelating compound of one of claims 1 through 3 and an element selected from the group consisting of Re, Al, In, As, Y, Cu, Tc, Er, Sn, Te, Pr, Ga, Pt, At, Au, Tb, Pd, Dy, Pm, Sm, Gd, Ho, Tm, Yb, Lu, Rh, Ag, F and I.

6. The complex according to claim 5, wherein the element is a radionuclide.

7. A pharmaceutical formulation comprising the chelating compound of claim 1 or the complex of claim 5 and a pharmaceutically acceptable carrier.

8. A chelator coupled pharmaceutical including the chelating compound of claim 1 or the complex of claim 5 being covalently coupled to a compound bearing a pharmacophore structure.

9. The chelator coupled pharmaceutical of claim 8, wherein the chelator pharmaceutical is a peptide conjugate of a peptide agonist or peptide antagonist, especially a SRIF antagonist, with the chelating compound of claim 1 or the complex of claim 5.

10. Use of the chelating compound of claim 1 or the complex of claim 5 for preparing a chelator coupled pharmaceutical, especially a peptide conjugate.

11. A method of preparing chelating compounds of Formula 10A and 10B including the step of:
conversion of compound 9A or 9B with compound 8 and subsequent remove of the benzyl protective moiety
